# EUROPÄISCHE PATENTSCHRIFT

(11) **EP 0 629 856 B1**
(45) Veröffentlichungstag und Bekanntmachung des Hinweises auf die Patenterteilung: **23.08.2000**
(21) Anmeldenummer: 94108762.9
(22) Anmeldetag: 08.06.1994
(51) Int. Cl.: G01N 33/543, G01N 33/58, G01N 33/53, C12Q 1/68

(54) **Verfahren zum Nachweis metabolisch markierter DNA**
Method for the detection of metabolically labelled DNA
Méthode pour la detection d'ADN métaboliquement marqué

(30) Priorität: 12.06.1993 DE 4319506
(43) Veröffentlichungstag der Anmeldung: 21.12.1994
(73) Patentinhaber: Roche Diagnostics GmbH, 68298 Mannheim (DE)
(72) Erfinder: Trauth, Bernhard, Dr., D-82362 Weilheim (DE); Hinzpeter, Matthias, Dr., D-80689 München (DE); Doppler, Clemens, Dr., D-82402 Seeshaupt (DE); Russmann, Eberhard, Dr., D-82377 Penzberg (DE)

(56) Entgegenhaltungen:
- EP-A- 0 628 818
- WO-A-91/15599
- WO-A-91/17263
- WO-A-92/17610
- FR-A- 2 660 925
- JOURNAL OF CLINICAL AND LABORATORY IMMUNOLOGY, Bd. 23, 1987, EDINBURGH GB, Seiten 153-159, XP000196007 F. MARTINON ET AL.:
- JOURNAL OF IMMUNOLOGICAL METHODS, Bd. 82, 1985, NEW YORK US, Seiten 169-179, XP000568765 T. PORSTMANN ET AL.:

## Beschreibung

Die Erfindung betrifft ein Verfahren zum nicht-radioaktiven Nachweis metabolisch markierter DNA sowie einen zur Durchführung dieses Verfahrens geeigneten Reagens-Kit.

Bei zellbiologischen Untersuchungen ist die Bestimmung der Proliferationsfähigkeit von Zellen von zentraler Bedeutung. Hierzu wird die DNA der zu untersuchenden Zellpopulation metabolisch markiert, d.h. den Zellen werden markierte Vorstufen der DNA-Biosynthese angeboten, die von proliferierenden Zellen in die DNA eingebaut werden. Bei der Standardmethode zur metabolischen Markierung von genomischer DNA wird als solche Vorstufe tritiummarkiertes Thymidin verwendet. Mit zunehmender Dosierung des tritiummarkierten Thymidins sowie zunehmender Markierungsdauer wird jedoch eine zunehmende Fragmentierung der markierten DNA beobachtet (E. Solary et al., Experimental Cell Research 203 (1992), 495 - 498). Die Herstellung von monoklonalen Antikörpern gegen das Thymidinanalogon 5-Brom-2'-desoxyuridin ermöglicht den Nachweis von nicht radioaktiv über den Einbau von Bromdesoxyuridin metabolisch markierter DNA (H. Gratzner, Science 218 (1982), 474 - 475). Obwohl zunächst auch ein monoklonaler Antikörper beschrieben wurde, welcher Bromdesoxyuridin in nativer, also nicht denaturierter DNA erkennen sollte (N. Gonchoroff et al., Cytometry 6 (1985), 506 - 512), wird in einer späteren, genaueren Untersuchung der Bindungseigenschaften von monoklonalen Antikörpern gegen Bromdesoxyuridin beschrieben, daß alle bislang bekannten monoklonalen Antikörper gegen Bromdesoxyuridin nur an ein Bromdesoxyuridin in einem Einzelstrangbereich binden (M. Miller et al., J. Immunol. 136 (1986), 1791 -1795). Bei Verwendung von Bromdesoxyuridin zur metabolischen Markierung von DNA ist deshalb für den Nachweis der markierten DNA eine Denaturierung der DNA für die Bindung des Antikörpers erforderlich. Bei den beschriebenen zellulären Testsystemen werden die Zellen durch Behandlung mit HCl/Ethanol fixiert und dabei gleichzeitig an die DNA gebundene Proteine abgelöst und die DNA denaturiert. Ein Nachteil dieser zellulären Testsysteme ist jedoch ein hoher unspezifischer Background sowie eine undefinierte Zugänglichkeit der metabolisch markierten DNA für den Antikörper, da das gesamte zelluläre Material fixiert wird. Damit sind ausreichend reproduzierbare quantitative Bestimmungen mit derartigen zellulären Testsystemen nicht möglich.

Von F. Martinon et al. (J. Clin. Lab. Immunol. 23 (1987), 153 - 159) wird ein Sandwich-ELISA zum Nachweis von mit Bromdesoxyuridin markierter DNA beschrieben, bei welchem ein erster Antikörper gegen Bromdesoxyuridin zur Immobilisierung der DNA verwendet wird und ein weiterer markierter Antikörper gegen Bromdesoxyuridin zum Nachweis der gebundenen DNA eingesetzt wird. Da hier bereits für die Bindung an die feste Phase die nachzuweisende metabolisch markierte DNA als Einzelstrang vorliegen muß, erfordert dieses Verfahren eine aufwendige Denaturierung der zu untersuchenden DNA in Lösung durch eine Säure- oder Basebehandlung oder einen Hitzedenaturierungsschritt vor der Bindung an die feste Phase. Damit ist dieses Verfahren für eine routinemäßige Bestimmung von metabolisch markierter DNA zu arbeits- und zeitaufwendig.

Aufgabe der Erfindung war es daher, ein Verfahren zum Nachweis metabolisch markierter DNA zur Verfügung zu stellen, welches leicht durchführbar ist und reproduzierbare quantitative Bestimmungen ermöglicht.

Diese Aufgabe wird gelöst durch ein Verfahren zum nicht-radioaktiven Nachweis metabolisch markierter DNA in einer Zellfraktion, welches dadurch gekennzeichnet ist, daß man
a) die Zellfraktion mit einem Antikörper, der an DNA bindet, inkubiert, der vor oder nach dieser Inkubation an eine feste Phase gebunden wird,
b) die DNA denaturiert,
c) einen markierten Antikörper gegen die metabolische Markierung zusetzt,
d) feste und flüssige Phase trennt und
e) die Markierung des Antikörpers aus Schritt c) in einer der beiden Phasen als Maß für die metabolisch markierte DNA bestimmt.

Mit dem erfindungsgemäßen Verfahren kann die in üblicher Weise zum Beispiel über den Einbau von Bromdesoxyuridin metabolisch markierte DNA auf einfache Weise unter reproduzierbaren Bedingungen nachgewiesen werden. Das erfindungsgemäße Verfahren vereinfacht daher die bekannten Verfahren zur Bestimmung der Proliferationsfähigkeit von Zellen, bei denen die Proliferationsfähigkeit über eine metabolische Markierung der DNA bestimmt wird. Der erfindungsgemäße einfache Nachweis einer metabolisch markierten DNA ist insbesondere auch zur Bestimmung der Zytotoxizität von chemischen Verbindungen, Antikörpern oder zytotoxischen Zellen von Bedeutung. Hierbei wird der Einfluß dieser Verbindungen bzw. Zellen auf die Proliferationsfähigkeit von Zellen bestimmt oder eine Schädigung der Zellen durch Nachweis der markierten DNA außerhalb des Zellkerns der zu untersuchenden Zellen bestimmt.

Die metabolische Markierung kann für den Nachweis über das erfindungsgemäße Verfahren durch den Einbau beliebiger Nukleosid-Analoga der natürlichen DNA-Biosynthese-Vorstufen erfolgen, sofern dieses Analogon wie eine natürliche Vorstufe in DNA eingebaut wird und ein Antikörper gegen dieses Analogon bekannt ist.

Zur Durchführung des erfindungsgemäßen Verfahrens wird zunächst ein Antikörper, der an DNA bindet, direkt oder indirekt an eine feste Phase gebunden. Diese Bindung erfolgt in dem Fachmann bekannter Art und Weise, z.B. über eine adsorptive Bindung des Antikörpers an die feste Phase oder durch Bindung eines biotinylierten Antikörpers, der an DNA bindet, an eine mit Streptavidin beschichtete feste Phase (Herstellung der mit Streptavidin beschichteten festen Phase z. B. gemäß EP-A 0 269 092). Als Antikörper, der an DNA bindet, kann jeder Antikörper verwendet werden, der direkt oder über einen weiteren Bindungspartner an DNA bindet. Antikörper, welche direkt an DNA binden, werden z.B. beschrieben in Andre-Schwartz et al., Clin. Immunol. Immunopathol. 31 (1984), 261. Daneben kann aber auch ein Antikörper gegen ein mit der DNA assoziiertes Protein, wie insbesondere ein Histon, verwendet werden, wenn eine metabolisch markierte DNA nachgewiesen werden soll, die mit einem solchen Protein assoziiert ist, d.h. es gebunden enthält. Jeder dieser Antikörper kann sowohl als komplettes Immunglobulin als auch als funktionelles Fragment eines Immunglobulins wie z.B. Fab oder F(ab')₂-Fragment verwendet werden. Die als Probe verwendete Zellfraktion kann entweder die Gesamt-DNA einer Zelle oder eine angereicherte DNA-Fraktion aus dem Zellkern oder dem Zytoplasma oder auch DNA-Fragmente aus dem Zellkulturüberstand von lysierten Zellen sein. Unter lysierten Zellen sind dabei solche Zellen zu verstehen, deren Zytoplasmamembran mindestens soweit aufgelöst ist, daß zelluläre DNA oder DNA-Fragmente aus der Zelle in den Zellkulturüberstand übertreten können. Derartige lysierte Zellen werden z.B. durch Behandlung mit einem Detergens, vorzugsweise einem nicht-ionischen Detergens erhalten oder durch Inkubation mit zytotoxischen T-Zellen oder Natural-Killer-Zellen. Die dabei erhaltenen DNA-Fragmente können ohne Vorbehandlung verwendet werden.

Die metabolische Markierung der DNA oder DNA-Fragmente erfolgt vorzugsweise über den Einbau von Bromdesoxyuridin. Als markierter Antikörper gegen die metabolische Markierung wird dann ein markierter Antikörper gegen Bromdesoxyuridin verwendet. Es hat sich überraschenderweise gezeigt, daß die für die Bindung des Antikörpers erforderliche Denaturierung der gebundenen DNA möglich ist, ohne daß sich die lediglich über einen Antikörper an die feste Phase gebundene DNA von der festen Phase löst. Die Denaturierung kann dabei in der dem Fachmann für eine Denaturierung in Lösung oder für zelluläre Testsysteme bekannten Art und Weise erfolgen, z.B. durch Behandlung mit Mikrowellen oder durch Zusatz einer Nuklease, vorzugsweise der Exonuklease III. Zur Denaturierung über Mikrowellenbehandlung wird der Reaktionsansatz für 0,5 bis 5 Minuten mit Mikrowellen einer Leistung von 500 bis 1000 W behandelt. Die Denaturierung über eine Nuklease erfolgt durch Zusatz von 4 bis 300 Units/ml Nuklease und Inkubation bei 37°C für 30 Minuten in einem Tris-Puffer (66 mmol/l Tris, 0,66 mmol/l MgCl₂ und 1 mmol/l 2-Mercaptoethanol) bei pH 7,5 bis 8,5, vorzugsweise pH 8,0.

Nach Inkubation der denaturierten DNA mit einem markierten Antikörper gegen Bromdesoxyuridin sowie Trennung von fester und flüssiger Phase kann dann die metabolische Markierung der DNA in einfacher Weise über die Markierung des Antikörpers bestimmt werden. Dieser Antikörper kann entweder direkt mit einem Enzym, einem Chemilumineszenz- oder Fluoreszenzfarbstoff markiert sein, oder z.B. durch einen entsprechend markierten weiteren Antikörper markiert werden.

Durch Verwendung eines Zellkulturüberstandes als Probelösung für das erfindungsgemäße Verfahren können speziell metabolisch markierte DNA-Fragmente von Zellen, welche infolge einer Schädigung, wie z.B. durch zytotoxische T-Zellen, Natural-Killer-Zellen, Makrophagen oder LAK-Zellen lysiert wurden, nachgewiesen werden (Berke, Immunol. Today 6 (1991) 21 - 27 und Berke, Immunol. Today 12 (1991), 396 - 399).

Ein bevorzugter Gegenstand der Erfindung ist daher eine Variante des erfindungsgemäßen Verfahrens, bei welcher als Zellfraktion der Zellkulturüberstand von lysierten Zellen zugegeben wird.

Im Gegensatz zu Zellen, welche durch zytotoxische T-Zellen oder Natural-Killer-Zellen lysiert werden, enthalten durch sogenannte Apoptose abgestorbene Zellen metabolisch markierte DNA-Fragmente nur im Zytoplasma. Erst bei längerer Inkubation (über 4 Stunden) treten auch bei apoptotischen Zellen infolge einer sekundären Schädigung (die Folge der langen Inkubationszeit ist) DNA-Fragmente in den Zellkulturüberstand über. Bei der Apoptose handelt es sich um einen aktiven Vorgang der eukaryontischen Zelle, welcher zum programmierten Zelltod führt oder Folge eines induzierten Zelltods ist, wie er z.B. durch chemische Verbindungen wie Camptothecin, Dioxine oder bestimmte Antikörper oder durch ionisierende Strahlung ausgelöst wird (Duvall et al., Immunol. Today 7 (1986), 115 - 119). Durch Verwendung einer zytoplasmatischen Zellfraktion können daher in einer weiteren Variante des erfindungsgemäßen Verfahrens speziell auch metabolisch markierte DNA-Fragmente apoptotischer Zellen nachgewiesen werden.

Ein besonders bevorzugter Gegenstand der Erfindung ist daher eine Variante des erfindungsgemäßen Verfahrens, bei welcher eine zytoplasmatische Zellfraktion mit dem Antikörper, der an DNA bindet, inkubiert wird. Die zytoplasmatische Zellfraktion wird dabei vorzugsweise durch Behandlung mit einem nicht-ionischen Detergens erhalten. Derartige Detergenzien sind dem Fachmann bekannt. Vorzugsweise wird NP-40 (Ethylphenylpolyethylenglycol), Tween 20 (Polyoxyethylensorbitanmonolaurat) oder Triton-X100 (Octylphenolpolyethylenglyolether) verwendet. Sie lysieren nur die Zytoplasmamembran, nicht jedoch die Kernmembran einer eukaryontischen Zelle. Da bei apoptotischen Zellen keine metabolisch markierte DNA in den Zellkulturüberstand übertritt, braucht der Zellkulturüberstand vor der Gewinnung der zytoplasmatischen Fraktion nicht abgetrennt zu werden. Als zytoplasmatische Zellfraktion kann daher auch ein Rohlysat, welches durch direkte Behandlung der Zellkultur mit den genannten Detergentien gewonnen wurde, verwendet werden.

Ein weiterer Gegenstand der Erfindung ist die Verwendung des erfindungsgemäßen Verfahrens zur Bestimmung der Wirkung oder Aktivität von Apoptose auslösenden Agenzien wie ionisierende Strahlen oder chemische Verbindungen wie Dioxine, bestimmte Antikörper oder Camptothecin.

Während die mit dem erfindungsgemäßen Verfahren nachweisbaren metabolisch markierten DNA-Fragmente bei Zellen, welche durch zytotoxische T-Zellen lysiert wurden, bereits nach kurzer Zeit in den Zellkulturüberstand freigesetzt werden, finden sich diese Fragmente bei über Apoptose abgestorbenen Zellen während den üblichen Inkubationszeiten nur in der zytoplasmatischen Zellfraktion (s. oben). Das erfindungsgemäße Verfahren eignet sich daher zur Unterscheidung von Zellen, welche durch zytotoxische T-Zellen lysiert wurden, gegenüber apoptotischen Zellen. Dazu wird zunächst die DNA der zu untersuchenden Zellkultur in bekannter Weise, vorzugsweise über den Einbau von Bromdesoxyuridin metabolisch markiert. Anschließend wird in zwei parallelen Ansätzen über das erfindungsgemäße Verfahren untersucht, ob sich metabolisch markierte DNA-Fragmente im Zellkulturüberstand oder nur in der zytoplasmatischen Zellfraktion nachweisen lassen. Zum Nachweis von metabolisch markierten DNA-Fragmenten in der Zytoplasmafraktion wird die zu untersuchende Zellkultur wie oben beschrieben mit einem nicht-ionischen Detergens behandelt. Nur in diesem Ansatz sind auch bei apoptotischen Zellen metabolisch markierte DNA-Fragmente im Zellkulturüberstand nachweisbar. In dem zweiten Parallelansatz ohne Detergensbehandlung sind dagegen nur bei Vorliegen von Zellen, welche durch zytotoxische T-Zellen lysiert wurden, metabolisch markierte DNA-Fragmente im Zellkulturüberstand nachweisbar.

Ein weiterer Gegenstand der Erfindung ist ein Reagens-Kit zum Nachweis metabolisch markierter DNA-Fragmente, enthaltend einen Antikörper, der an DNA bindet, sowie einen Antikörper gegen die metabolische Markierung. Vorzugsweise wird als Antikörper gegen die metabolische Markierung ein Antikörper gegen Bromdesoxyuridin verwendet.

Ein bevorzugter Gegenstand der Erfindung ist daher ein Reagens-Kit zum Nachweis metabolisch markierter DNA-Fragmente, enthaltend einen Antikörper, der an DNA bindet, sowie einen Anti-Bromdesoxyuridin-Antikörper.

Ein besonders bevorzugter Gegenstand der Erfindung ist ein erfindungsgemäßer Reagens-Kit, bei welchem der Antikörper, der an DNA bindet, zur Bindung an eine feste Phase in biotinylierter Form vorliegt und welcher zusätzlich eine mit Streptavidin beschichtete feste Phase enthält.

Ein weiterer Gegenstand der Erfindung ist schließlich ein erfindungsgemäßer Reagens-Kit, welcher zusätzlich eine Nuklease zur Denaturierung der antikörpergebundenen DNA enthält. Als Nuklease wird dabei vorzugsweise Exonuklease III verwendet.

Die Erfindung wird durch die folgenden Beispiele näher erläutert.

### Beispiel 1

### Nachweis metabolisch markierter DNA-Fragmente

Die zu untersuchenden Zellen (z.B. HL60-Zellen, ATCC CCL 240) werden zunächst durch Inkubation mit 0,01 mmol/l Bromdesoxyuridin für 18 h bei +37°C metabolisch markiert. Nach Entfernung des überschüssigen, nicht in die DNA inkorporierten Bromdesoxyuridins durch Waschen mit Kulturmedium werden die Zellen (jeweils 200 µl, Zellzahl siehe Tabelle 1) mit 2 µg/ml Camptothecin (Sigma, München) in einer Mikrotiterplatte bei 37°C für 4 h inkubiert. Danach werden je Ansatz 20 µl 10-fach konzentrierter Lysepuffer (PBS/50 mmol/l EDTA / 2 % Tween 20) zur Lyse zugegeben, die Mikrotiterplatte zentrifugiert und 100 µl Überstand je Vertiefung für den Nachweis entnommen (PBS = 137 mmol/l NaCl; 2,7 mmol/l KCl; 8 mmol/l Na₂HPO₄ und 1,5 mmol/l KH₂PO₄).

Zum Nachweis der metabolisch markierten DNA wird eine weitere Mikrotiterplatte durch 100 mmol/l NaCO₃-Puffer pH 9,6 mit einem Antikörper gegen DNA (5 µg/ml, Boehringer Mannheim GmbH, Ident.-Nr. 1525760) beschichtet. Anschließend wird die metabolisch markierte DNA durch Zugabe von 100 µl Überstand (siehe oben) je Vertiefung und 90 min Inkubation bei Raumtemperatur an den Wandantikörper gebunden. Nach Waschen mit PBS wird die restliche Flüssigkeit aus den Vertiefungen der Mikrotiterplatte z.B. durch Ausklopfen entfernt. Danach wird die gebundene doppelsträngige DNA durch Mikrowellenbehandlung (5 min, 650 Watt, AEG Micromat 275Z) oder alternativ durch Zugabe von 100 µl (entsprechend 1 Unit je Vertiefung der Mikrotiterplatte) Exonuklease III (Boehringer Mannheim GmbH, Katalog-Nr. 779 717) denaturiert bzw. partiell abgebaut. Der Ansatz, bei welchem die DNA durch Mikrowellenbehandlung denaturiert wurde, wird anschließend für 10 Minuten bei 8°C im Kühlschrank abgekühlt. Der mit Exonuklease III behandelte Ansatz wird anschließend dreimal mit PBS gewaschen. Anschließend wird in beiden Ansätzen jeweils 100 µl/Vertiefung eines peroxidasemarkierten Antikörpers gegen Bromdesoxyuridin (Boehringer Mannheim GmbH, Katalog-Nr. Ident.-Nr. 1 449 338, 250 mU/ml) zugegeben und gebundener Antikörper und damit markierte DNA-Fragmente über die Peroxidasereaktion mit ABTS® als Substrat (100 µl je Vertiefung, Boehringer Mannheim GmbH, Katalog-Nr. 1204521) und Messung der Extinktion bei 405 nm bestimmt. Die folgende Tabelle 1 zeigt die gute Korrelation des erhaltenen Meßsignals mit der Menge an metabolisch markierter DNA sowohl bei Denaturierung über Mikrowellenbehandlung als auch bei Denaturierung durch Nukleasebehandlung.

**Tabelle 1**

| Nachweis metabolisch markierter DNA-Fragmente | | |
|---|---|---|
| Anzahl vormarkierter Zellen | E (405 nm) bei Denaturierung mit 1 Unit Nuklease pro Vertiefung | E (405 nm) bei Denaturierung über Mikrowelle |
| 6 x 10³ | 0,870 | 0,752 |
| 3 x 10³ | 0,572 | 0,420 |
| 1,5 x 10³ | 0,348 | 0,257 |
| 7,5 x 10² | 0,195 | 0,148 |
| 3,75 x 10² | 0,099 | 0,077 |

### Beispiel 2

### Nachweis metabolisch markierter DNA-Fragmente im Kulturüberstand von Zellen, welche durch zytotoxische T-Zellen lysiert wurden

Zellen der murinen P815-Zellinie (ATCC TIB 64) werden wie in Beispiel 1 beschrieben mit Bromdesoxyuridin metabolisch markiert. Nach Auswaschen überschüssigen Bromdesoxyuridins werden jeweils 2 x 10⁴ Zellen (100 µl) mit unterschiedlichen Mengen von Allogen-stimulierten zytotoxischen T-Zellen (Effektor zu Targetzellverhältnisse siehe Tabelle 2) in einer Mikrotiterplatte bei 37°C für 4 h inkubiert (die Herstellung der zytotoxischen T-Zellen erfolgt gemäß Current Protocols in Immunology, eds. J. Coligan, A. Kruisbeek, D. Margulies, E. Shevach und W. Strober, John Wiley and Sons, New York (1992), Chapter 3.11). Danach wird die Mikrotiterplatte zentrifugiert und jeweils 100 µl Überstand je Ansatz für die Bestimmung verwendet, die wie in Beispiel 1 beschrieben über eine Denaturierung durch Mikrowellenbehandlung erfolgt.

Die nachfolgende Tabelle zeigt, daß die Menge an in Zellkulturüberstand bestimmbarer metabolisch markierter DNA mit der Menge an zugesetzten Effektorzellen und damit mit dem Ausmaß der zytotoxischen Aktivität korreliert. Im Zellkulturüberstand der ohne Effektorzellen inkubierten P815 Targetzellen sowie im Zellkulturüberstand der zytotoxischen Effektorzellen ist dagegen keine metabolisch markierte DNA nachweisbar.

**Tabelle 2**

| Nachweis metabolisch markierter DNA-Fragmente im Zellkulturüberstand von Zellen, welche durch zytotoxische T-Zellen lysiert wurden | | |
|---|---|---|
| Verhältnis der zytotoxischen T-Zellen zu Target-Zellen | E (405 nm) zytotoxische T-Zellen plus Targetzellen¹ | E (405 nm) zytotoxische T-Zellen ohne Targetzellen² |
| 12 : 1 | 0,977 | 0,010 |
| 6 : 1 | 1,092 | 0,007 |
| 3 : 1 | 0,894 | 0,0 |
| 1,5 : 1 | 0,752 | 0,009 |
| 0,75 : 1 | 0,620 | 0,001 |
| 0,4 : 1 | 0,464 | 0,0 |

| | | |
|---|---|---|
| ¹⁾ Im Zellkulturüberstand der identischen Menge an P815-Targetzellen, die in Abwesenheit von zytotoxischen T-Zellen inkubiert wurden, konnte ebenfalls keine signifikante Menge einer metabolisch markierten DNA nachgewiesen werden (E (405 nm) 0,073). | | |
| ²⁾ Als Kontrolle wurde der Zellkulturüberstand einer entsprechenden Menge von zytotoxischen T-Zellen, die ohne vormarkierte Targetzellen inkubiert wurden, mitgeführt. | | |

### Beispiel 3

### Nachweis metabolisch markierter DNA-Fragmente im Zytoplasma apoptotischer Zellen

Zunächst werden HL60-Zellen (ATCC CCL 240) wie in Beispiel 1 angegeben mit Bromdesoxyuridin metabolisch markiert. Nach Auswaschen von überschüssigem Bromdesoxyuridin werden die Zellen mit unterschiedlichen Konzentrationen von Camptothecin (5000 bis 0 ng/ml) in einer Mikrotiterplatte bei 37°C für 3 bzw. 4 h inkubiert. Anschließend wird die Mikrotiterplatte zentrifugiert, ein Aliquot des Zellkulturüberstands (100 µl/Vertiefung) für den Nachweis metabolisch markierter DNA im Zellkulturüberstand gemäß Beispiel 2 abgenommen und die in der Mikrotiterplatte verbliebenen Zellen durch Zugabe von 100 µl/Vertiefung 2fach konzentriertem Lysepuffer (PBS / 10 mmol/l EDTA / 0,4 % Tween 20) lysiert. Der Nachweis der metabolisch markierten DNA in dem so erhaltenen Lysat erfolgt dann wie in Beispiel 1 beschrieben.

Tabelle 3 zeigt für den 3 h lang inkubierten Ansatz, daß bei apoptotischen Zellen metabolisch markierte DNA nur in der zytoplasmatischen Fraktion (also erst nach Behandlung mit einem Detergens im Überstand) nachweisbar ist, nicht jedoch im Zellkulturüberstand.

Tabelle 4 zeigt für den 4 h lang inkubierten Ansatz, daß die die Menge dieser zytoplasmatischen metabolisch markierten DNA-Fragmente mit der Menge an Camptothecin, also der eine Apoptose auslösenden Aktivität, korreliert.

**Tabelle 3**

| Nachweis metabolisch markierter DNA-Fragmente bei apoptotischen Zellen | | |
|---|---|---|
| Fraktion | E (405 nm) Kontrolle (0 ng/ml Camptothecin) | E (405 nm) Apoptose (200 ng/ml Camptothecin) |
| Zellkulturüberstand (gemäß Beispiel 2) | 0,011 | 0,020 |
| Zytoplasma (Lysat nach Detergensbehandlung) | 0,068 | 0,770 |

**Tabelle 4**

| Nachweis metabolisch markierter DNA-Fragmente im Zytoplasma apoptotischer Zellen | |
|---|---|
| Camptothecin (ng/ml) | E (405 nm) |
| 5000 | 1,677 |
| 1000 | 1,457 |
| 200 | 1,006 |
| 40 | 0,625 |
| 8 | 0,342 |
| 1,6 | 0,295 |
| 0 | 0,265 |

## Patentansprüche

1. Verfahren zum nicht-radioaktiven Nachweis metabolisch markierter DNA in einer Zellfraktion, dadurch gekennzeichnet, daß man
a) die Zellfraktion mit einem Antikörper, der an DNA bindet, inkubiert, der vor oder nach dieser Inkubation an eine feste Phase gebunden wird,
b) die DNA denaturiert,
c) einen markierten Antikörper gegen die metabolische Markierung zusetzt,
d) feste und flüssige Phase trennt und
e) die Markierung des Antikörpers aus Schritt c) in einer der beiden Phasen als Maß für die metabolisch markierte DNA bestimmt.

2. Verfahren zum Nachweis von über den Einbau von Bromdesoxyuridin metabolisch markierter DNA gemäß Anspruch 1, dadurch gekennzeichnet, daß als markierter Antikörper ein markierter Antikörper gegen Bromdesoxyuridin verwendet wird.

3. Verfahren gemäß einem der Ansprüche 1 oder 2, dadurch gekennzeichnet, daß die Denaturierung der DNA über eine Mikrowellenbehandlung oder über eine Nukleasebehandlung erfolgt.

4. Verfahren gemäß einem der Ansprüche 1 bis 3, dadurch gekennzeichnet, daß man den Antikörper, der an DNA bindet, zur Bindung an die feste Phase biotinyliert und eine mit Streptavidin beschichtete feste Phase verwendet.

5. Verfahren gemäß einem der Ansprüche 1 bis 4, dadurch gekennzeichnet, daß als Zellfraktion der Zellkulturüberstand von lysierten Zellen zugegeben wird.

6. Verfahren gemäß einem der Ansprüche 1 bis 4, dadurch gekennzeichnet, daß als Zellfraktion die Zytoplasmafraktion einer Zelle verwendet wird.

7. Verwendung eines Verfahrens gemäß einem der Ansprüche 1 bis 4 oder 6 zur Bestimmung der Wirkung oder Aktivität von Apoptose auslösenden Agenzien wie ionisierende Strahlen, chemischen Verbindungen wie Dioxinen, Antikörpern oder Camptothecin sowie von zytotoxischen Zellen, Natural-Killer-Zellen, Makrophagen oder LAK-Zellen.

8. Reagens-Kit zum Nachweis metabolisch markierter DNA-Fragmente, enthaltend einen Antikörper, der an DNA bindet, sowie einen Antikörper gegen die metabolische Markierung.

9. Reagens-Kit gemäß Anspruch 8, dadurch gekennzeichnet, daß der Antikörper gegen die metabolische Markierung ein Anti-Bromdesoxyuridin-Antikörper ist.

10. Reagens-Kit gemäß einem der Ansprüche 8 oder 9, dadurch gekennzeichnet, daß der Antikörper, der an DNA bindet, zur Bindung an eine feste Phase in biotinylierter Form vorliegt und der Kit zusätzlich eine mit Streptavidin beschichtete feste Phase enthält.

11. Reagens-Kit gemäß einem der Ansprüche 8 bis 10, dadurch gekennzeichnet, daß er zusätzlich eine Nuklease zur Denaturierung von antikörpergebundener DNA enthält.

## Claims

1. Method for the non-radioactive detection of metabolically labelled DNA in a cell fraction, wherein
a) the cell fraction is incubated with an antibody which binds to DNA and this antibody is bound before or after incubation to a solid phase,
b) the DNA is denatured,
c) a labelled antibody against the metabolic label is added,
d) the solid and liquid phase are separated and
e) the label of the antibody from step c) is determined in one of the two phases as a measure of the metabolically labelled DNA.

2. Method for the detection of metabolically labelled DNA by means of bromodeoxyuridine incorporation as claimed in claim 1, wherein a labelled antibody against bromodeoxyuridine is used as the labelled antibody.

3. Method as claimed in one of the claims 1 or 2, wherein the denaturation of the DNA is carried out by means of microwave treatment or by nuclease treatment.

4. Method as claimed in one of the claims 1 to 3, wherein the antibody which binds to DNA is a biotinylated for binding to the solid phase and a solid phase coated with streptavidin is used.

5. Method as claimed in one of the claims 1 to 4, wherein the cell culture supernatant of lysed cells is added as the cell fraction.

6. Method as claimed in one of the claims 1 to 4, wherein the cytoplasmic fraction of a cell is used as the cell fraction.

7. Use of a method as claimed in one of the claims 1 to 4 or 6 for the determination of the effect or activity of agents causing apoptosis such as ionizing radiation, chemical compounds such as dioxins, antibodies or camptothecin as well as of cytotoxic cells, natural killer cells, macrophages or LAK cells.

8. Reagent kit for the detection of metabolically labelled DNA fragments containing an antibody that binds to DNA as well as an antibody against the metabolic label.

9. Reagent kit as claimed in claim 8, wherein the antibody against the metabolic label is an anti-bromodeoxyuridine antibody.

10. Reagent kit as claimed in one of the claims 8 or 9, wherein the antibody that binds to DNA is present in a biotinylated form for binding to a solid phase and the kit additionally contains a solid phase coated with streptavidin.

11. Reagent kit as claimed in one of the claims 8 to 10, wherein it additionally contains a nuclease for denaturing antibody-bound DNA.

## Revendications

1. Procédé pour la détection non radioactive d'ADN métaboliquement marqué dans une fraction cellulaire, caractérisé en ce que
a) on incube la fraction cellulaire avec un anticorps qui se lie à l'ADN, l'anticorps étant lié à une phase solide avant ou après cette incubation,
b) on dénature l'ADN,
c) on ajoute un anticorps marqué contre le marqueur métabolique,
d) on sépare la phase solide et la phase liquide, et
e) on détermine le marquage de l'anticorps à partir de l'étape c) dans une des deux phases comme mesure pour l'ADN métaboliquement marqué.

2. Procédé pour la détection d'ADN métaboliquement marqué via incorporation de la bromodésoxyuridine selon la revendication 1, caractérisé en ce qu'on utilise, à titre d'anticorps marqué, un anticorps marqué contre la bromodésoxyuridine.

3. Procédé selon la revendication 1 ou 2, caractérisé en ce que la dénaturation de l'ADN a lieu via un traitement par micro-ondes ou via un traitement à la nucléase.

4. Procédé selon l'une quelconque des revendications 1 à 3, caractérisé en ce qu'on soumet l'anticorps qui se lie à l'ADN à une biotinylation à des fins de liaison à la phase solide et on utilise une phase solide enduite de streptavidine.

5. Procédé selon l'une quelconque des revendications 1 à 4, caractérisé en ce qu'on ajoute, à titre de fraction cellulaire, le produit surnageant de la culture de cellules lysées.

6. Procédé selon l'une quelconque des revendications 1 à 4, caractérisé en ce qu'on utilise, à titre de fraction cellulaire, la fraction cytoplasmique d'une cellule.

7. Utilisation d'un procédé selon l'une quelconque des revendications 1 à 4 ou 6 pour déterminer l'effet ou l'activité d'agents déclenchant une apoptose, tels que des rayons ionisants, des composés chimiques tels que les dioxines, des anticorps ou la camptothécine, de même que de cellules cytotoxiques, de cellules tueuses naturelles, de macrophages ou de cellules LAK.

8. Nécessaire de réactif pour la détection de fragments d'ADN métaboliquement marqués contenant un anticorps qui se lie à l'ADN, ainsi qu'un anticorps contre le marqueur métabolique.

9. Nécessaire de réactif selon la revendication 8, caractérisé en ce que l'anticorps contre le marqueur métabolique est un anticorps antibromodésoxyuridine.

10. Nécessaire de réactif selon la revendication 8 ou 9, caractérisé en ce que l'anticorps, qui se lie à l'ADN, est présent sous forme biotinylée à des fins de liaison à une phase solide et le nécessaire contient en outre une phase solide enduite de streptavidine.

11. Nécessaire de réactif selon l'une quelconque des revendications 8 à 10, caractérisé en ce qu'il contient en outre une nucléase pour la dénaturation de l'ADN lié à un anticorps.
